# EUROPEAN PATENT APPLICATION

(11) **EP 0 674 908 A1**
(43) Date of publication of application: **04.10.1995**
(21) Application number: 95104389.2
(22) Date of filing: 24.03.1995
(51) Int. Cl.: A61L 27/00

(54) **Collagen implants having improved tensile properties**

(30) Priority: 29.03.1994 US 219319
(71) Applicant: COLLAGEN CORPORATION, Palo Alto, California 94303 (US)
(72) Inventor: Chu, George H., Cupertino, CA 95014 (US); Rao, Prema R., Los Gatos, CA 95032 (US)
(74) Representative: Schwan, Gerhard, Dipl.-Ing.

(57) **Abstract**

Collagen-based implants, e.g. cartilage implants, having improved tensile strength, elongation and flexibility are disclosed. The implants are prepared by forming a mixture of an atelopeptide collagen fiber and a crosslinking agent, forming the mixture into a gelled shaped article, drying the gelled shaped article under controlled drying conditions to a moisture level of 30 to 40% by weight, and curing the dried article under controlled curing conditions to a moisture level of less than 30% by weight. The drying and curing conditions give rise to tightly crosslinked, rehydratable implants having an equilibrium hydration value of less than 350% by weight.

## Description

### BACKGROUND OF THE INVENTION

### Field of the Invention

The invention relates to the preparation of collagen-based implants for tissue repair, particularly cartilage repair, having improved tensile properties and elasticity.

### Description of Related Art

Collagen is the major protein component of bone, cartilage, skin and connective tissue in animals. Collagen in its native form is typically a rigid, rod-shaped molecule approximately 300 nm long and 1.5 nm in diameter. It is composed of three collagen polypeptides which form a tight triple helix. The collagen polypeptides are characterized by a long midsection having the repeating sequence- Gly-X-Y-, where X and Y are often proline or hydroxyproline, bounded at each end by the "telopeptide" regions, which constitute less than about 5% of the molecule. The telopeptide regions of the collagen chains are typically responsible for the cross-linking between chains, and for the immunogenicity of the protein. Collagen occurs in several types, having differing physical and chemical properties. The most abundant types are Types I-III.

Collagen is typically isolated from natural sources, such as bovine hide, cartilage, or bones. Bones are usually dried, defatted, crushed, and demineralized to extract collagen, while hide and cartilage ore usually minced and digested with proteolytic enzymes (other than collagenase). As collagen is resistant to most proteolytic enzymes, this procedure conveniently serves to remove most of the contaminating protein found with collagen.

Luck et al, U.S. Pat. No. 4,488,911 discloses a method for preparing collagen in solution (CIS) wherein native collagen is extracted from animal tissue in dilute aqueous acid, followed by digestion with an enzyme such as pepsin, trypsin or pronase. The enzyme digestion removes the telopeptide portions of the collagen molecules, providing "atelopeptide" collagen in solution. The atelopeptide CIS so produced is substantially nonimmunogenic, and is also substantially non-cross-linked due to loss of the primary crosslinking regions. The CIS may then be precipitated to produce collagen fibers which resemble native collagen fibers. The precipitated, reconstituted fibers may additionally be cross-linked using a chemical agent (for example aldehydes such as formaldehyde and glutaraldehyde), or using heat or radiation. The resulting products are suitable for use in medical implants due to their biocompatability and reduced immunogenicity.

The prior art discloses numerous applications involving the use of collagen to form membranes or implants for medical use. For example, US Patent 4,725,671 discloses a process for preparing membranes suitable for use in tissue repair or as implants wherein an acidic solution of collagen, alone or admixed with a suspension of cross-linked collagen, is treated with a buffering agent to form a fibrous suspension which is permitted to gel. The gel is then compressed to remove or absorb the aqueous fraction from the gel to form a fibrous membrane which is subsequently dried in air at room temperature.

U.S. Patent 5,123,925 discloses a method for preparing an implant suitable for treatment of bony defects comprising forming a mixture of a calcium phosphate mineral powder and a reconstituted fibrillar atelopeptide collagen, shaping the mixture, drying the mixture under dry flowing air at 35-45° C to a moisture content of less than 1%, rehydrating the dried structure until a moisture content of 1-6% is achieved, and subjecting the rehydrated structure to sterilizing radiation.

U.S. Patent 5,246,457 discloses bone repair compositions having improved compressive modulus and rupture strength based on a mixture of mineral powder and less than 10% by weight of fibrillar collagen formed by mixing, molding and drying (lyophilizing) the composition. The composition may be crosslinked prior to drying and optionally heat cured after drying by heating at 60°-120°C for a period of 4-168 hours, or heat cured in the moist state at 37°C for 1-10 days, followed by drying at 37°C.

While these and other prior art disclosures describe general methods for the processing and treatment of collagen-containing compositions to optimize the efficacy of implants and membranes for their intended uses, there is still a need to provide collagen structural implant materials having a higher elasticity and toughness (tensile strength) such as would render these materials more effective as tendon or cartilage implants.

### SUMMARY OF THE INVENTION

This invention provides a process for the drying and curing of an at least partially crosslinked fibrillar collagen matrix to provide a cartilage-like implant material having enhanced tensile strength, flexibility and elasticity. The process comprises the following general steps:
a) forming an aqueous mixture comprising fibrillar collagen and a water soluble crosslinking agent therefor;
b) forming said mixture into a shaped gelled article;
c) drying said shaped gelled article at a temperature in the range of from about 0°C to about 45°C under conditions of drying such that the weight loss per hour, based on starting weight, ranges from about 1 to about 50% by weight and until said article has a moisture content in the range of from about 30 to about 40% by weight; and
d) curing said dried article by heating under inert gas or vacuum at a temperature in the range of from greater than 45°C up to about 120°C for a period of time of about 1 to about 48 hours until said article has a moisture content below about 30% by weight.

It has been found that implants, e.g., rod-shaped articles, prepared in accordance with the controlled drying/curing process of this invention provide dense, hard articles having much improved tensile properties in the dry stage and a reduced amount of volume swelling when rehydrated to the equilibrium wet stage, thereby providing cartilage-like implants having high crosslink density and improved tensile strength, flexibility and elasticity.

### DETAILED DESCRIPTION OF THE INVENTION

Fibrillar collagen material which may be employed in preparing the structures of this invention includes collagen reconstituted from a collagen solution such as that marketed by Collagen Corporation under the trade name VITROGEN^{R} 100. This material is a solution containing about 2-4 mg./ml. of bovine atelopeptide collagen at a pH of about 2.0. The collagen may be insolubilized into fibrillar form by neutralization using about 10% by weight of a 0.2M Na₂HPO₄/0.09M Na0H buffer, pH 11.2.

In a more preferred embodiment, the collagen employed is already in the fibrillar form such as the dispersions available from the Collagen Corporation under the trade name ZYDERM^{R} Collagen Implant (ZCI). These are fibrillar collagen dispersions in saline solution generally containing from 30 to 70 mg of atelopeptide collagen per mL of solution.

The collagen source may also include glutaraldehyde crosslinked atelopeptide fibers (GAX) which have been subjected to interfiber crosslinking reactions such as a material prepared in accordance with the teachings of U.S. Patent 4,582,640. This kind of material is also available from the Collagen Corporation under the trade name ZYPLAST^{R} Collagen Implant.

Yet another collagen source includes modified collagen, e.g., methylated or succinylated collagen such as prepared by treating isolated collagen with a solution of methanolic hydrochloric acid or succinic anhydride at 4 to 40°C for 0.5 to 168 hours, followed by washing.

Mixtures of these various collagen materials may also be utilized.

In a first step of the process of this invention, the collagen in the form of a fibrillar dispersion is mixed with an aqueous solution (buffered or non-buffered) of a suitable cross linking agent capable of forming interfiber crosslinks between the collagen fibers. Suitable crosslinking agents include difunctional polymers prepared by reacting a dicarboxylic anhydride (e.g., glutaric or succinic anhydride) with a diol such as polyethylene or polypropylene glycol having a molecular weight of about 400 to 10,000 daltons, preferably from about 500 to 5,000 daltons, to form a polymer derivative (e.g., succinate), which may then be activated by esterification with a convenient leaving group, e.g., N-hydroxysuccinimide, N,N'-disuccinimidyl oxalate, N,N'-disuccinimidyl carbonate and the like. Preferred dicarboxylic anhydrides used to form the crosslinking compositions include glutaric anhydride, adipic anhydride, succinic anhydride, 1,8-naphthalene dicarboxylic anhydride and the like. These materials and their method of preparation are more particularly disclosed in U.S. Patent 5,162,430, the complete disclosure of which is incorporated herein by reference.

Other suitable crosslinking agents include the more conventional materials such as aldehydes. Suitable aldehydes are formaldehyde, glutaraldehyde, acid aldehyde, glyoxal, glyoxal pyruvic aldehyde, and aldehyde starch.

The amount of crosslinking agent mixed with the collagen dispersion is generally in excess, ranging from about a 3-10 molar excess of crosslinking agent to collagen solids on a molar basis.

Crosslinking of the fibrillar collagen is initiated by forming an intimate mixture of about 100 parts per weight of the collagen aqueous dispersion (which contains from about 10 to about 100 mg/ml of collagen) with from about 5 to about 100 parts per weight of aqueous crosslinking solution (containing from about 1 to about 20% by weight of dissolved crosslinking agent). More preferably the collagen aqueous dispersion contains from about 20 to 50 mg/ml of collagen, and the aqueous crosslinking solution contains from about 4 to about 10% by weight of dissolved crosslinking agent.

After mixing, the composition is cast or extruded to form a desired shape and permitted to incubate to form a gelled shaped article. Suitable shapes include sheets, tubes cylinders, cords or ropes. In the case of tendon or ligament implants, it is preferable to form rods or filaments for weaving into cords or ropes, which may be prepared in a block or cylindrical shaped mold or hypodermic needle.

Incubation of the shaped article takes place over a period of from 5 to 30 hours, more preferably 16 to 20 hours at a temperature in the range of 20 to 45°C, more preferably 35 to 40°C. The resulting incubation product is an at least partially crosslinked, gelled product.

The gelled collagen is removed from the mold after incubation and may optionally be washed several times using a phosphate buffer solution to remove excess, unreacted crosslinking agent. Preferably, however, the product is not washed at this stage in order to permit additional crosslinking to occur during the product drying and curing stages.

The drying and curing conditions to which the gelled article is subjected are critical with respect to the achievement of hard, dense implants which exhibit enhanced tensile properties and improved flexibility and elongation when rehydrated. Drying is conducted by subjecting the article to a constant flow of air or inert gas (helium or nitrogen) in an oven or other suitable container at a temperature in the range of from about 0°C to about 45°C, more preferably from about 20°C to 40°C, under conditions of drying such that the weight loss per hour ranges from about 10 to about 30% by weight. After a period of from about 3 to 10 hours, a dried product having a moisture content in the range of from about 30 to about 40% by weight is obtained. Generally speaking, the dried product at this stage will have a weight and volume in the range of from about 5-10%, more preferably from about 6 to 8%, of the weight and volume of the gelled article prior to drying.

Curing of the resulting dried product is conducted by heating the product in an oven at a temperature in the range of greater than 45°C up to about 120°C and maintaining the article in that temperature range under vacuum or under a constant flow of inert gas for a period of time of from about 1 to about 48 hours until the article has a moisture content below 30% by weight, more preferably below about 25% by weight, and most preferably in the range of from about 10 - 25% by weight. In the more preferred embodiment, curing is effected by heating at about 80°C to about 100°C for a period of from about 10 to 24 hours, most preferably from about 15 to 20 hours.

The resulting cured, shaped article is a dense, hard material exhibiting superior tensile strength as compared with similar articles which are prepared by simple air drying of the gelled article at temperatures of 20-45°C down to a moisture content of less than 40% by weight. The tensile strength of the cured article at this stage generally will range from about 35 to 45 Newton/mm².

Prior to implantation, the cured articles prepared in accordance with this invention are rehydrated in water to the equilibrium water uptake and may be washed at this time to remove any unreacted crosslinking agent and any other impurities which may be present. The rehydrated implant may also be sterilized using gamma radiation at this stage by techniques well known in the art, such as disclosed in U.S. Patent 5,123,925.

The rehydrated implants prepared in accordance with this invention exhibit improved toughness, flexibility and elongation properties making them ideally suited for tendon or ligament repair. The combination of drying and curing steps set forth herein give rise to a more tightly crosslinked product which exhibits minimum swelling after equilibrium hydration is reached. The equilibrium rehydration value, expressed as increase of weight from the dried, cured stage to the hydration stage, is generally less than 350% by weight, preferably in the range of 250-300% by weight. Generally speaking, the implant is rehydrated to the extent that its weight and volume after hydration will fall in the range of from about 15 - 25%, more preferably from about 20 - 25%, of the weight and volume of the gelled article prior to drying.

Growth and bioactive factors to aid healing or regrowth of normal tissue may also be included in the implants prepared according to this invention. These may be added to the collagen dispersion prior to gellation or may be infused into the dried implant at the time of rehydration. These factors include transforming growth factors (TGF-alpha or TGF-beta), ptalelet derived growth factors, connective tissue activating peptides and similar factors as disclosed in U.S. Patents 5,024,841 and 5,162,430.

Compositions of the invention may be prepared in a form that is dense and rigid enough to substitute for cartilage. These compositions are useful for repairing and supporting tissue which require some degree of structure, for example in reconstruction of the nose, ear, knee, larynx, tracheal rings, and joint surfaces. One can also replace tendon, ligament and blood vessel tissue using appropriately formed cartilaginoid material. In these applications, the material is generally cast or molded into shape. In the case of tendons and ligaments, it may be preferable to form filaments for weaving into cords or ropes. In the case of artificial blood vessels it may be advantageous to incorporate a reinforcing mesh (e.g., nylon or the like).

Compositions of the invention are also useful for coating articles for implantation or relatively long term residence within the body. Such surface treatment renders the object nonimmunogenic, and reduces the incidence of foreign body reactions. Accordingly, one can apply compositions of the invention to catheters, cannulas, bone protheses, cartilage replacement, breast implants, minipumps and other drug delivery devices, artificial organs, and the like. Application may be accomplished by dipping the object into the reaction mixture while crosslinking is occurring, drying and curing as set forth herein. One may pour or otherwise apply the reaction mixture if dipping is not convenient. Alternatively, one may use flexible sheets or membranous forms of collagen-polymer conjugate to wrap the object with, sealing corners and edges with the reaction mixture.

In another embodiment, the object may be dipped in a viscous fibrillar collagen solution until the object is completely coated. The collagen solution is fixed to the object by dipping the collagen-coated object into a dPEG* (pH 7) solution bath, and then drying and curing as set forth herein.

The following examples are illustrative of the invention.

### Example 1

This example details the preparation of an activated difunctional polyethylene glycol-based crosslinking agent. Difunctional PEG 3400 (34g, 10 mmol, Aldrich Chemical Co.) is dissolved in 1,2-dichoroethane (250 mL) and heated at reflux with glutaric anhydride (10g) and pyridine (4 mL) under nitrogen for 3 days. The solution is then filtered and the solvent evaporated, and the residue dissolved in water (100 mL) and washed with diethyl ether (2x50 mL). The resulting PEG diglutarate is extracted from the water with chloroform (2x50 mL), and the chloroform evaporated to yield PEG-diglutarate. The PEG-diglutarate is then dissolved in DMF (200 mL) at 37⁰C., and N-hydroxysuccinimide (10% molar xs) added. The solution is cooled to 0⁰C., and an equivalent amount of dicyclohexylcarbodiimide added in DMF solution (10 mL). The mixture is left at room temperature for 24 hours, and then filtered. Cold benzene (100 mL) is then added, and the PEG-di(succinimidyl glutarate) (dPEG-SG) precipitated by adding petroleum ether (200 mL) at 0⁰C. The precipitate is collected on a sintered glass filter. Dissolution in benzene, followed by precipitation with petroleum ether is repeated three times to provide "activated" dPEG.

### Example 2

This example details the preparation of a cured collagen implant in accordance with this invention.

26.1 mL of a fibrillar collagen dispersion at a 35 mg/ml concentration (pH 7.2) was transferred into a 60cc syringe. 293 mg of dPEG as prepared in Example 1 was dissolved in 2.9 mL of a phosphate buffer solution (pH 7.2) and the solution was transferred to a second 60cc syringe. The two components were then mixed by back and forth motion between the syringes, joined by a Luerlock connector, to form a homogeneous solution, which was then poured into a tube shaped mold for incubation. The mold was incubated at 37⁰C for a period of 18 hours, after which time a firm collagen gel was removed from the mold.

The firm collagen gel was dried at 37°C to a moisture content of 34.8% by weight. At this stage, the weight and volume of the dry material reached about 8% of its original weight and volume before drying. The tensile strength of the dried product was 39.1 Newton/mm², with a peak melting temperature of 60.2°C measured in hydrated form by Differential Scanning Calorimetry. Subsequently, the dried material was cured at 80°C under vacuum overnight. The cured material had a moisture content of 24.6% and a tensile strength of 41.6 Newton/mm², and a peak melting temperature of 62.8°C measured in hydrated form by Differential Scanning Calorimetry.

The dried and cured material was rehydrated in phosphate buffered saline (PBS) to reach equilibrium after 30 min. at room temperature. The moisture content for this material was 75.7% and the weight and volume of this material was about 23% of the original weight and volume before drying.

Testing methods used for the above measurements were as follows:
1. Tensile Strength: Using Instron Model 4202, and 5KN tensile load cell. Sample was glued on to tape at each side with Crazy Glue, then the grips hold tapes. The force required to break the sample in the middle was measured. The force was normalized by the cross section of the sample. The results were presented in Newton/mm².
2. Differential Scanning Calorimetry (DSC): peak Melting temperature of each sample was measured by a Mettler Differential Scanning Calorimetry Instrument TA3000.

The above-described preferred embodiments of the present invention are not intended to limit the scope of the present invention as demonstrated by the claims which follow, as one skilled in the art can, with minimal experimentation, extend the disclosed concepts of the invention to the claimed scope of the invention.

## Claims

1. A process for preparing a collagen-based implant material having improved tensile strength comprising:
a. forming an aqueous mixture comprising a fibrillar collagen and a water soluble crosslinking agent therefor;
b. forming said mixture into a gelled shaped article;
c. drying said gelled shaped article at a temperature in the range of from about 0⁰C to about 45⁰C under conditions of drying such that the weight loss per hour, based on starting weight, ranges from about 1 to about 50% by weight, and until said article has a moisture content in the range of from about 30 to about 40% by weight; and
d. curing said dried article by heating under inert gas or vacuum at a temperature in the range of from greater than 45⁰C up to about 120⁰C for a period of time of about 1 to about 48 hours until said article has a moisture content below 30% by weight.

2. The process of claim 1 wherein the weight loss per hour in step (c) ranges from about 10 to about 30% by weight.

3. The process of claim 1 wherein the drying temperatures in step (c) ranges from about 20°C to about 40°C.

4. The process of claim 1 wherein the curing temperature in steps (d) ranges from about 80⁰C to about 100⁰C.

5. The process of claim 1 wherein the curing time in step (d) ranges from about 10 to about 24 hours.

6. The process of claim 1 wherein the moisture content of said article from step (d) is below about 25% by weight.

7. The process of claim 1 wherein said cured article exhibits an equilibrium rehydration value of less than about 350% by weight.

8. The process claim 7 wherein said equilibrium rehydration value is less than about 300% by weight.

9. The process of claim 8 wherein said equilibrium hydration value lies in the range of from about 250 to 300% by weight.

10. The process of claim 1 wherein the dried article from step (c) exhibits a weight and volume in the range of from about 5-10% of the weight and volume of said gelled article prior to drying.

11. The process of claim 1 wherein said cured article from step (d) is contacted with water and rehydrated such that the weight and volume of said rehydrated article falls in the range of from about 15-25% of the weight and volume of said gelled article prior to drying.

12. A collagen-based implant having a tensile strength of about 35 to about 45 Newton/mm², said implant prepared by the process of claim 1.
